# EUROPÄISCHE PATENTANMELDUNG

(11) **EP 4 389 024 A1**
(43) Veröffentlichungstag der Anmeldung: **26.06.2024**
(21) Anmeldenummer: 22216318.0
(22) Anmeldetag: 23.12.2022
(51) Int. Cl.: A61B 17/32

(54) **ULTRASCHALLSYSTEM FÜR DIE ULTRASCHALLCHIRURGIE UND VERFAHREN ZU DESSEN BEDIENUNG**

(71) Anmelder: Söring GmbH, 25451 Quickborn (DE)
(72) Erfinder: Neumann, Florian, 22459 Hamburg (DE); Buschschlüter, Steffen, 25485 Hemdingen (DE); Maltzen, Gesa, 23619 Heilshoop (DE)
(74) Vertreter: Lobemeier, Martin Landolf

(57) **Zusammenfassung**

Ultraschallsystem für die Ultraschallchirurgie mit einem eine Sonotrode und einen piezoelektrischen Wandler aufweisenden Ultraschall-Instrument, einem den Wandler antreibenden Ultraschall-Generator und einer mit dem Ultraschall-Instrument kommunizierenden Einrichtung zum Zuführen einer Spülflüssigkeit zum Ultraschallinstrument mit einer vorbestimmten Spülrate und/oder zum Bewirken eines vorbestimmten für die Absaugung von mittels dem Ultraschall-Instrument fragmentiertem Gewebe eingerichteten am Ultraschall-Instrument anliegenden Unterdrucks, gekennzeichnet durch einen in Abhängigkeit des Gewebetyps zwischen einer vorbestimmten minimalen Abtragrate und einer vorbestimmten maximalen Abtragrate eine aktuelle Abtragrate einstellenden Regler (20), wobei die Abtragraten jeweils durch ein aus Sonotroden-Amplitude einerseits und Spülrate oder Unterdruck andererseits bestehendes Wertepaar oder ein aus Sonotroden-Amplitude, Spülrate und Unterdruck bestehendes Wertetripel bestimmt sind.

## Beschreibung

Die Erfindung betrifft ein für die Ultraschallchirurgie eingerichtetes Ultraschallsystem und ein Verfahren zu dessen Bedienung. Insbesondere betrifft die Erfindung ein Ultraschallsystem für die Ultraschallchirurgie mit einem eine Sonotrode und einen piezoelektrischen Wandler aufweisenden Ultraschall-Instrument, einem den Wandler antreibenden Ultraschall-Generator und einer mit dem Ultraschall-Instrument kommunizierenden Einrichtung zum Zuführen einer Spülflüssigkeit zum Ultraschallinstrument mit einer vorbestimmten Spülrate und/oder zum Bewirken eines vorbestimmten für die Absaugung von mittels dem Ultraschall-Instrument fragmentiertem Gewebe eingerichteten am Ultraschall-Instrument anliegenden Unterdrucks. Daneben betrifft die Erfindung speziell ein Verfahren zum Betreiben eines solchen Ultraschallsystems mittels Erzeugen einer vorbestimmten Sonotroden-Amplitude, Zuführen einer Spülflüssigkeit zum Ultraschallinstrument mit einer vorbestimmten Spülrate und/oder Bewirken eines vorbestimmten für das Absaugen von mittels dem Ultraschall-Instrument fragmentiertem Gewebe eingerichteten am Ultraschall-Instrument anliegenden Unterdrucks, zum Bewirken einer vorbestimmten Abtragrate.

In der Ultraschallchirurgie, speziell in der Leberchirurgie, der Neurochirurgie und der Wirbelsäulenchirurgie, kommen für den Grobabtrag und die Feinpräparation von Gewebe Ultraschallsysteme zum Einsatz. Die bekannten, für die Ultraschallchirurgie eingerichteten Ultraschallsysteme bestehen insbesondere aus einem Ultraschall-Generator der Wechselstrom mit einer sehr hohen Frequenz an ein mit einer Einrichtung zum Spülen und/oder Saugen versehenes Ultraschall-Instrument überträgt. Das Ultraschall-Instrument wandelt die elektrische Energie mittels Piezokeramiken in mechanische Bewegung um, welche über eine Sonotrode verstärkt werden. Bei Kontakt zwischen schwingender Sonotrode und Gewebe kommt es je nach technischer Konstruktion zu einem Gewebeeffekt in Form von Fragmentierung, Ablation, Koagulation oder Dissektion. Das Ausmaß dieser Effekte hängt einerseits vom Energieeintrag der Sonotrode und andererseits von den akustischen und biologischen Eigenschaften des Gewebes ab, welche sich letztendlich in deren mechanischen Eigenschaften niederschlagen. Weiches Gewebe (z.B. Leberparenchym) erfordert einen geringeren Energieeintrag als stark elastisches (z.B. Blutgefäße) oder hartes Gewebe (z.B. Knochen).

In dieser Abhängigkeit des Gewebeeffektes vom Gewebetyp besteht einerseits der Vorteil bei der Verwendung von Ultraschallsystemen in der Ultraschallchirurgie. Die chirurgische Behandlung kann bei Verwendung von Ultraschall-Instrumenten auch in unmittelbarer Nähe zu hochsensiblen Strukturen erfolgen. Andererseits besteht dadurch kein strenger Zusammenhang zwischen den vom Operateur bestimmten Parametern und dem Ausmaß des Gewebeeffektes.

Nachteilig ist an den bekannten Systemen daher grundsätzlich, dass eine erfolgreiche chirurgische Behandlung einen hinreichenden Erfahrungsschatz des Operateurs bei der Bedienung der bekannten Ultraschallsysteme voraussetzt. In der klinischen Praxis arbeiten Chirurginnen und Chirurgen mit einer Bandbreite von *"Gewebeentfernungsgeschwindigkeiten*", die im technischen Sinn als Abtrag- oder Dissektionsrate verstanden werden kann. In der Nähe von kritischen Strukturen, wie zum Beispiel Blutgefäßen, wird langsam reseziert, um das Risiko von Komplikationen zu minimieren. Grundsätzlich soll die Resektion aber auch in einer akzeptablen Zeit mit einem akzeptablen Aufwand durchgeführt werden, um die Operationsdauer und die Belastung für den Patienten gering zu halten. Einerseits existiert also eine untere Grenze (*"Mindestabtragrate"*) für den gewünschten Gewebeeffekt, andererseits soll aber auch zügig, wenngleich nicht unkontrolliert reseziert werden, sodass für den gewünschten Gewebeeffekt gleichzeitig eine obere Grenze ("*Maximalabtragrate"*) existiert.

Die Einstellung der vom Operateur angestrebten "*Gewebeentfernungsgeschwindigkeit*" bzw. Abtragrate wird vom Operateur erfahrungsgemäß bzw. intuitiv in Abhängigkeit des *in situ* vorgefundenen Operationsfelds bei Verwendung eines Ultraschall-Instruments mit vorgegebener Ultraschallfrequenz (im Bereich von 20 kHz bis 60 kHz) am Ultraschall-Generator vorgenommen. Hierfür stehen dem Operateur als Parameter die Amplitude der Sonotrode (in % des Maximalausschlags; auch als *"Ultrasound"* bezeichnet), die Spülrate (*"Irrigation"*) in ml/min und/oder der die Absaugung bestimmende Unterdruck (*"Aspiration"*) in mbar zur Verfügung.

Die Abhängigkeit der Geräteparameter von der konkreten Situation während der Operation macht dadurch eine wiederholte Anpassung der einzelnen Einstellungen äußerst komplex und erfordert ein hohes Maß an Erfahrung und Intuition. Die zur Verfügung stehenden Einstellungskombinationen erfordern eine Lernkurve und die Einstellungswahl im unsterilen Bereich setzt eine reibungslose Kommunikation und Zusammenarbeit mit weiteren Mitarbeitern im OP voraus. Tatsächlich existieren bei den bekannten Ultraschallsystemen mehrere tausend Kombinationsmöglichkeiten für die Einstellungen, von denen ein Großteil nicht zu den gewünschten Gewebeeffekten führt.

Aufgabe der Erfindung ist es daher, ein für die Ultraschallchirurgie eingerichtetes Ultraschallsystem zu schaffen, das eine einfache, sichere und effiziente Bedienung ermöglicht, sowie ein Verfahren zu dessen Bedienung. Insbesondere soll eine intuitive und effektive Bedienung eines Ultraschallsystems auch bei geringer Erfahrung ermöglicht sein, wobei der Schwerpunkt auf der einfachen Bedienung liegt, bei der auch der unerfahrene Operateur sein Augenmerk auf das Operationsfeld lenken kann, ohne sich permanent um die Auswahl und Einstellung von Parametern kümmern zu müssen.

Diese Aufgabe wird erfindungsgemäß durch das Ultraschallsystem mit den Merkmalen von Anspruch 1 und das Verfahren zum Betreiben des Ultraschallsystems mit den Merkmalen von Anspruch 7 gelöst. Die Unteransprüche geben jeweils vorteilhafte Ausgestaltungen der Erfindung wieder.

Grundgedanke der Erfindung ist es, ein Ultraschallsystem und ein Verfahren zum Betreiben eines Ultraschallsystems bereitzustellen, durch die der Anwender, also der Operateur, sich stärker auf das Operationsfeld konzentrieren kann und weniger Einstellungen am Ultraschallsystem vornehmen muss, um einen bestimmten Effekt zu erzielen. Der Bediener muss nunmehr zunächst noch das zu bearbeitende Gewebe anhand seiner mechanischen Eigenschaften kategorisieren. Die mechanischen Eigenschaften können sich beispielhaft am Elastizitätsmodul des Gewebetyps orientieren. Danach muss im Verlauf der Operation lediglich die Effektstärke geregelt werden, die sich aus Sonotroden-Amplitude, Spülrate und/oder Unterdruck ergibt und einer Abtragrate (oder Dissektionsrate) entspricht. Die mit dem Stellglied "Effektstärke" verknüpften, aus Sonotroden-Amplitude, Spülrate und/oder Unterdruck bestehenden Parameter werden für den Bediener automatisch an die Effektstärke angepasst, sodass dieser sich nicht mehr um die Auswahl der einzelnen in Wechselbeziehung zueinanderstehenden Parameter kümmern muss. Die Bedienung des Ultraschallsystems ist somit stark vereinfacht, sodass nicht nur erfahrene Operateure, sondern auch Operateure mit geringer Erfahrung von der Erfindung profitieren, wobei Fehler vermieden und die Versorgung des Patienten auf hohem Niveau sichergestellt werden kann.

Erfindungsgemäß wird also ein Ultraschallsystem für die Ultraschallchirurgie mit einem eine Sonotrode und einem piezoelektrischen Wandler aufweisenden Ultraschall-Instrument, einem den Wandler antreibenden Ultraschall-Generator und einer mit dem Ultraschall-Instrument kommunizierenden Einrichtung zum Zuführen einer Spülflüssigkeit zum Ultraschallinstrument mit einer vorbestimmten Spülrate und/oder zum Bewirken eines vorbestimmten für die Absaugung von mittels dem Ultraschall-Instrument fragmentiertem Gewebe eingerichteten am Ultraschall-Instrument anliegenden Unterdrucks vorgeschlagen, bei dem ein in Abhängigkeit eines vorbestimmten Gewebetyps zwischen einer vorbestimmten minimalen Abtragrate und einer vorbestimmten maximalen Abtragrate eine aktuelle Abtragrate einstellender Regler vorgesehen ist, wobei die Abtragraten jeweils durch ein aus Sonotroden-Amplitude einerseits und Spülrate oder Unterdruck andererseits bestehendes Wertepaar oder ein aus Sonotroden-Amplitude, Spülrate und Unterdruck bestehendes Wertetripel bestimmt sind.

So kann ein erfindungsgemäß ausgestaltetes Ultraschallsystem beispielsweise derart ausgestaltet sein, dass dieses für nur einen bestimmten Gewebetyp ausgelegt ist, wobei das über den Regler einzustellende Wertepaar oder Wertetripel von diesem Gewebetyp abhängig ist.

Bevorzugt weist das Ultraschallsystem jedoch eine Eingabeeinrichtung zur Auswahl eines vorbestimmten Gewebetyps aus einer Gruppe vorbestimmter Gewebetypen auf. Die der jeweiligen Effektstärke, die mittels des Reglers eingestellt wird, zugewiesenen Parameter des Wertepaars oder des Wertetripels unterscheiden sich in Abhängigkeit des vorbestimmten bzw. ausgewählten Gewebetyps.

Beispielsweise kann der Anwender zwischen einem weichen und einem harten Gewebetyp, gegebenenfalls mit Zwischenstufen mittlerer Härte unterscheiden, die insbesondere Elastizitätsmodulen entsprechen, speziell im Bereich von 830 +/- 64 Pa, 1840 +/- 180 Pa, 5862 +/- 1101 Pa und 12257 +/- 2708 Pa. Die für den gewählten Gewebetyp entsprechenden Kombinationen von Sonotroden-Amplitude, Spülrate und Unterdruck, die durch den Regler gemeinsam eingestellt werden, führen je nach mittels des Reglers eingestellter Effektstärke zu einer niedrigen oder einer hohen Abtragrate.

Insbesondere ist die Stellgröße des Reglers jeweils mit den Regelgrößen Sonotroden-Amplitude, Spülrate und Unterdruck monoton verknüpft, wobei die Regelgrößen Sonotroden-Amplitude, Spülrate und Unterdruck höchst bevorzugt jeweils einer eigenen monotonen Funktion folgen.

Ein Wertepaar kann insbesondere aus den Werten der Sonotroden-Amplitude und der Spülrate oder den Werten der Sonotroden-Amplitude und des Unterdrucks gebildet sein. Das Wertetripel besteht aus den über den Regler miteinander verknüpften Werten der Sonotroden-Amplitude, der Spülrate und des Unterdrucks. Es versteht sich, dass gegebenenfalls weitere Parameter mit den vorgenannten Parametern verknüpft sein können, die genannten Parameter jedoch für die Ausführung der Erfindung essentiell sind.

Eine für den Anwender (und den Patienten) besonders sichere Ausgestaltung des Ultraschallsystems wird dadurch erreicht, dass zusätzlich ein die Stellgröße des Reglers auf eine vorbestimmte Abtragrate begrenzender Schalter vorgesehen ist, wobei die vorbestimmte Abtragrate niedriger als die vorbestimmte maximale Abtragrate ist. Durch diese Ausgestaltung wird mit einem einfachen Schalter sichergestellt, dass die Abtragrate innerhalb eines vorbestimmten Sicherheitsbereichs liegt, der nicht - jedenfalls bei ansonsten vom Anwender angewendeter Methodik - durch den Regler überschritten werden kann. Speziell ist vorgesehen, dass die Einstellungen dieses *"Safety-Level"* bei den unterschiedlichen Gewebetypen jeweils die klinisch geforderte Mindest-Abtragrate von 15 mg/s erfüllen.

Ebenso wird erfindungsgemäß ein Verfahren zum Betreiben eines ein Ultraschall-Instrument mit einer Sonotrode und einem piezoelektrischem Wandler, einen den Wandler antreibenden Ultraschall-Generator und eine mit dem Ultraschall-Instrument kommunizierende Einrichtung, die zum Zuführen einer Spülflüssigkeit zum Ultraschallinstrument mit einer vorbestimmten Spülrate und/oder zum Bewirken eines vorbestimmten für die Absaugung von mittels dem Ultraschall-Instrument fragmentiertem Gewebe eingerichteten am Ultraschall-Instrument anliegenden Unterdrucks eingerichtet ist, aufweisenden Ultraschallsystems für die Ultraschallchirurgie mittels Erzeugen einer vorbestimmten Sonotroden-Amplitude, Zuführen einer Spülflüssigkeit zum Ultraschallinstrument mit einer vorbestimmten Spülrate und/oder Bewirken eines vorbestimmten für das Absaugen von mittels dem Ultraschall-Instrument fragmentiertem Gewebe eingerichteten am Ultraschall-Instrument anliegenden Unterdrucks, zum Bewirken einer vorbestimmten Abtragrate vorgeschlagen, bei dem die Sonotroden-Amplitude und die Spülrate und/oder der Unterdruck mittels eines einzigen Stellglieds in Abhängigkeit eines vorbestimmten Gewebetyps miteinander verknüpft sind.

Der vorbestimmte Gewebetyp ist insbesondere durch das Elastizitätsmodul des Gewebes bestimmt, wobei der vorbestimmte Gewebetyp insbesondere vom Gerätehersteller vorgegeben sein oder vom Anwender ausgewählt werden kann.

Wie zuvor für das System beschrieben kann zwischen einem weichen und einem harten Gewebetyp, gegebenenfalls mit Zwischenstufen mittlerer Härte ausgewählt werden, die insbesondere Elastizitätsmodulen von 830 +/- 64 Pa, 1840 +/- 180 Pa, 5862 +/- 1101 Pa und 12257 +/- 2708 Pa entsprechen. Die auf der Basis des gewählten Gewebetyps entsprechenden Kombinationen von Sonotroden-Amplitude, Spülrate und/oder Unterdruck, die durch das einzige Stellglied gemeinsam eingestellt werden, führen je nach mittels des Stellglieds eingestellter Effektstärke zu einer niedrigen oder einer hohen Abtragrate.

Das Betreiben des Ultraschall-Instruments erfolgt insbesondere durch Einstellen des Stellglieds auf ein aus Sonotroden-Amplitude, Spülrate und/oder Unterdruck bestehendes Wertepaar oder Wertetripel, das eine vorbestimmte Abtragrate bewirkt, die zwischen einer vorbestimmten minimalen Abtragrate und einer vorbestimmten maximalen Abtragrate liegt.

Die Sonotroden-Amplitude, die Spülrate und/oder der Unterdruck sind speziell mit dem Stellglied jeweils monoton verknüpft, wobei die Sonotroden-Amplitude, die Spülrate und/oder der Unterdruck besonders bevorzugt über den Regelbereich des Stellglieds jeweils einer eigenen monotonen Funktion folgen.

Im Folgenden wird die Erfindung anhand von in den beigefügten Zeichnungen dargestellten, besonders bevorzugt ausgestalteten Ausführungsbeispielen näher erläutert. Es zeigen:
- Fig. 1: eine Bedieneinheit eines ersten besonders bevorzugt ausgestalteten Ultraschallsystems nach der Erfindung, das für chirurgische Eingriffe an Knochen konzipiert ist;
- Fig. 2: eine Bedieneinheit eines zweiten besonders bevorzugt ausgestalteten Ultraschallsystems nach der Erfindung, das für endoskopische neurochirurgische Eingriffe vorgesehen ist; und
- Fig. 3: eine Bedieneinheit eines dritten besonders bevorzugt ausgestalteten Ultraschallsystems nach der Erfindung, das für offene neurochirurgische Eingriffe eingerichtet ist.

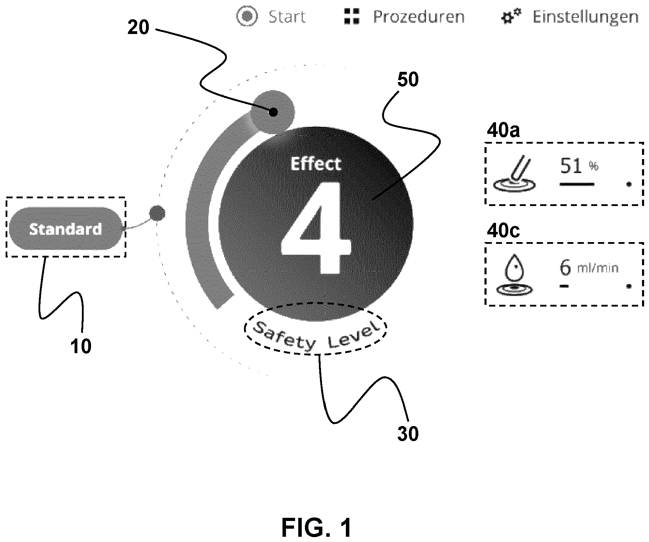

Fig. 1 zeigt eine Bedieneinheit eines besonders bevorzugt ausgestalteten Ultraschallsystems nach der Erfindung, das für chirurgische Eingriffe an Knochen konzipiert ist. Insbesondere zeigt Fig. 1 eine als Touchscreen ausgebildete Bedieneinheit 100 eines besonders bevorzugt ausgestalteten Ultraschallsystems nach der Erfindung.

Die Bedieneinheit 100 ist speziell für ein Ultraschallsystem für die Ultraschallchirurgie an Knochen mit einem eine Sonotrode aufweisenden Ultraschall-Instrument, einem das Instrument antreibenden Ultraschall-Generator und einer mit dem Ultraschall-Instrument kommunizierenden Spüleinrichtung ausgelegt. Das ultraschall-assistierte Knocheninstrument ist ein für die Dissektion von Knochen entwickeltes Instrument mit dem Wirbel im Rahmen von Dekompressionen, Stabilisierungs- oder Versteifungsoperationen dissektiert werden. Der Knochen wird mittels des Systems nicht zermahlen und abgetragen (wie mit dem Knochendrill), sondern wird durchschnitten und kann in Stücken entfernt werden. Störende Osteophyten werden daher nicht abgesaugt, sondern im Ganzen entnommen, bleiben vital und können dem Patienten wieder eingesetzt werden. Aufgrund der feineren, aber auch langsameren Arbeitsweise, zeichnet sich das Knocheninstrument durch geringe Temperatursteigerungen aus. Zur Kühlung wird das Instrument gespült. Je nach Lokalisation, Indikation und Entfernung zu Nerven passt der Bediener die mit der Dissektion verbundene Schnittgeschwindigkeit an. Die Schnittgeschwindigkeit bzw. die für den Schnitt notwendige Anpresskraft ist dabei von der Schwingungsamplitude abhängig. Da mit steigender Amplitude auch die thermische Belastung steigt, wird je nach Risiko-Einschätzung des Bedieners zugleich die Spülrate angepasst.

Das in Fig. 1 dargestellte System ist also auf einen vorbestimmten Gewebetyp, nämlich Knochen ausgelegt, der insbesondere durch ein vorbestimmtes Elastizitätsmodul bestimmt ist.

Die Bedieneinheit 100 gibt den vorbestimmten Gewebetyp im gezeigten Beispiel durch die Anzeige 10 mit "Standard" wieder, die vom Bediener im Normalfall nicht geändert werden kann.

Zur Auswahl steht dem Bediener allein die Effektstärke 50, die der Abtrag- bzw. Dissektionsrate entspricht, deren Betrag wiederum monoton mit der Sonotroden-Amplitude 40a und der Spülrate 40c verknüpft ist. Mittels des Reglers 20 steuert der Bediener also allein die Höhe des Effekts, wobei die Sonotroden-Amplitude 40a und die Spülrate 40c in Abhängigkeit vom vorbestimmten Gewebetyp automatisch geregelt werden. Der Bediener mag dabei mittels des mit "Safety Level" bezeichneten Schalters 30 die maximale Effektstärke bzw. Abtragrate auf einen Wert begrenzen, der unterhalb der für den vorbestimmten Gewebetyp vorgesehenen maximalen Abtragrate liegt.

Das hiermit vorgeschlagene Bedienkonzept kombiniert also die Parameter Sonotroden-Amplitude und Spülrate abhängig vom vorbestimmten Gewebetyp und abhängig von der gewünschten Schnittgeschwindigkeit unter Optimierung der thermischen Belastung für das umliegende Gewebe.

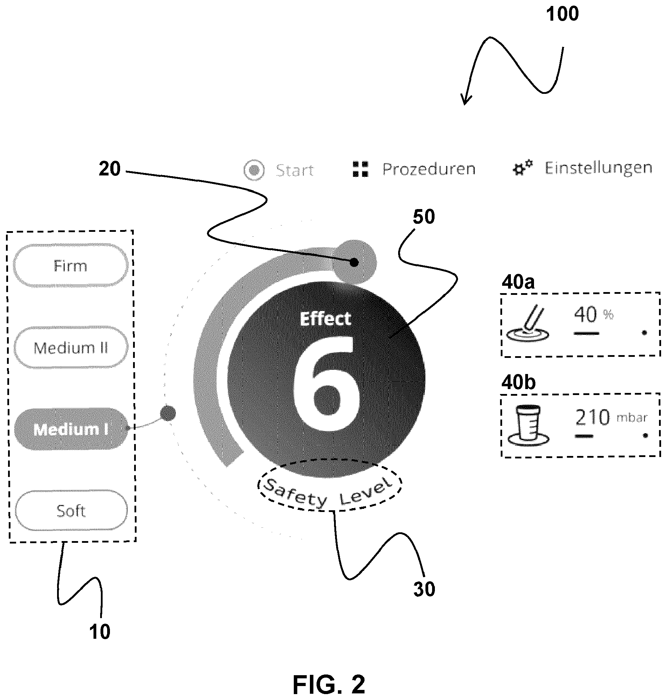

Fig. 2 zeigt eine Bedieneinheit eines besonders bevorzugt ausgestalteten Ultraschallsystems nach der Erfindung, das für endoskopische neurochirurgische Eingriffe vorgesehen ist. Wie zuvor zeigt auch Fig. 2 eine als Touchscreen ausgebildete Bedieneinheit 100 eines besonders bevorzugt ausgestalteten Ultraschallsystems nach der Erfindung.

Insbesondere werden mittels dieses Systems Tumore und Zysten im flüssigkeitsgefüllten Ventrikelsystem abgetragen. Die gewünschte Abtragsgeschwindigkeit richtet sich dabei - wie in der offenen Neurochirurgie - nach der Risikoeinschätzung und der gewünschten Abtragsgeschwindigkeit, die wiederum von der Gewebekonsistenz abhängig sind. Der Steuerung von Spülung und Absaugung kommt dabei eine besondere Bedeutung zu, da ein Unterdruck im Schädel zu einem Ventrikelkollaps führen kann. Grundsätzlich soll jede abgesaugte Flüssigkeitsmenge direkt durch Flüssigkeitszufuhr ersetzt werden. Die Flüssigkeitszufuhr findet daher nicht über einen aktiven Pumpmechanismus statt, sondern über eine Gravitationsspülung, die an das Endoskop angeschlossen ist. Die Spülung wird daher nicht über das Instrument oder den Generator gesteuert, womit lediglich die Parameter Sonotroden-Amplitude 40a und Absaugdruck 40b für das neue Bedienkonzept verbleiben.

Diese beiden Parameter sind abhängig von der vom Bediener in der Eingabeeinrichtung 10 auszuwählenden Gewebekonsistenz und der gewünschten Effektstärke 50 mittels des Reglers 20 miteinander kombiniert.

Auch in diesem Fall mag der Bediener mittels des mit "Safety Level" bezeichneten Schalters 30 die maximale Effektstärke bzw. Abtragrate auf einen Wert begrenzen, der unterhalb der für den vorbestimmten Gewebetyp vorgesehenen maximalen Abtragrate liegt.

Jedenfalls kann sich der Operateur im vorliegenden Fall nach Auswahl des vom Operateur zu bearbeitenden Gewebetyps auf das Operationsfeld konzentrieren und muss mittels des Reglers 20 lediglich die Effektstärke 50 anpassen, um eine Veränderung der Parameter Sonotroden-Amplitude 40a und Unterdruck 40b hervorzurufen, die mit dem Regler unter Berücksichtigung ausschließlich vorteilhafter Kombinationen monoton verknüpft sind.

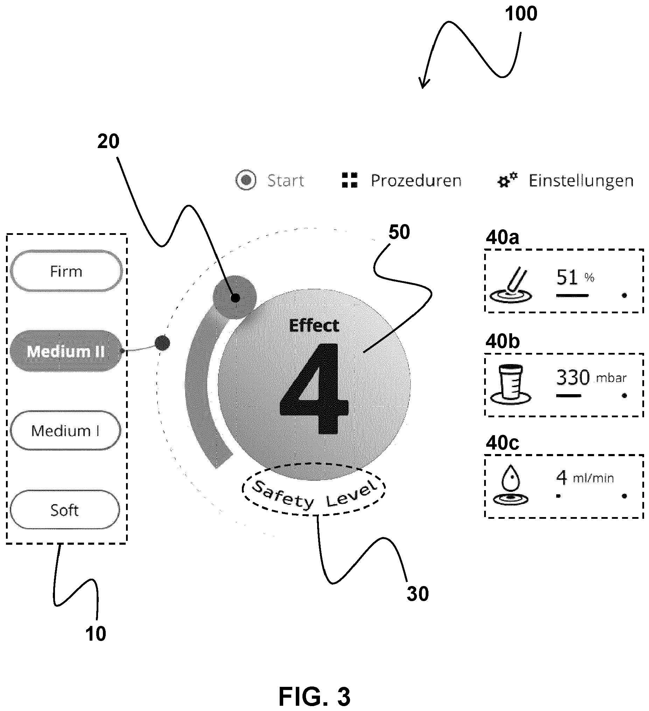

Fig. 3 zeigt schließlich die Bedieneinheit eines besonders bevorzugt ausgestalteten Ultraschallsystems nach der Erfindung, das für den Einsatz in der offenen Neurochirurgie vorgesehen ist. Insbesondere zeigt Fig. 3 eine als Touchscreen ausgebildete Bedieneinheit 100 eines erfindungsgemäß ausgestalteten Ultraschallsystems.

Die Bedieneinheit 100 ist speziell für ein Ultraschallsystem für die Neurochirurgie mit einem eine Sonotrode aufweisenden Ultraschall-Instrument, einem das Instrument antreibenden Ultraschall-Generator und einer mit dem Ultraschall-Instrument kommunizierenden Spül-Saug-Einrichtung ausgelegt. Die Bedieneinheit 100 weist eine Eingabeeinrichtung 10 zur Auswahl eines durch das Elastizitätsmodul eines mit dem Ultraschall-Instrument zu bearbeitenden Gewebes bestimmten Gewebetyps auf, das im gezeigten Beispiel als "Soft", "Medium I", "Medium II" und "Firm" bestimmt ist. Die Bezeichnungen "Soft", "Medium I", "Medium II" und "Firm" entsprechen dabei beispielsweise Elastizitätsmodulen von 830 +/-64 Pa, 1840 +/- 180 Pa, 5862 +/- 1101 Pa und 12257 +/- 2708 Pa.

Hat der Bediener den mittels des Ultraschallsystems zu bearbeitenden Gewebetyp festgelegt, wählt der Bediener die in der Anzeige 50 dargestellte Effektstärke mittels des Reglers 20 aus. Eine geringe Effektstärke entspricht dabei einer für den gewählten Gewebetyp geringen Abtragrate und eine hohe Effektstärke einer hohen Abtragrate in Bezug auf den gewählten Gewebetyp.

Der Bediener mag dabei mittels des mit "Safety Level" bezeichneten Schalters 30 die Effektstärke bzw. Abtragrate auf einen Wert begrenzen, der unterhalb der für den Gewebetyp vorgesehenen maximalen Abtragrate liegt.

Jedenfalls werden die Parameter Sonotroden-Amplitude, Spülrate und Unterdruck, die gemeinsam mit dem Regler 20 in Abhängigkeit vom mittels der Eingabeeinrichtung 10 gewählten Gewebetyps in den Anzeigen 40a, 40b, 40c angezeigt, sodass für den Bediener eine Korrelation zwischen der abstrakten Effektstärke und den die Abtragrate bestimmenden Parametern unmittelbar hergestellt ist.

Nach einer besonders bevorzugten Ausgestaltung der Erfindung kann zusätzlich vorgesehen sein, sodass der Operateur die Steuerung mittels des Reglers 20 abschalten und unmittelbar sowie unabhängig voneinander Veränderungen an der Sonotroden-Amplitude, der Spülrate und dem Unterdruck vornehmen kann.

## Patentansprüche

1. Ultraschallsystem für die Ultraschallchirurgie mit einem eine Sonotrode und einen piezoelektrischen Wandler aufweisenden Ultraschall-Instrument, einem den Wandler antreibenden Ultraschall-Generator und einer mit dem Ultraschall-Instrument kommunizierenden Einrichtung zum Zuführen einer Spülflüssigkeit zum Ultraschallinstrument mit einer vorbestimmten Spülrate und/oder zum Bewirken eines vorbestimmten für die Absaugung von mittels dem Ultraschall-Instrument fragmentiertem Gewebe eingerichteten am Ultraschall-Instrument anliegenden Unterdrucks,
**gekennzeichnet durch**
einen in Abhängigkeit eines vorbestimmten Gewebetyps zwischen einer vorbestimmten minimalen Abtragrate und einer vorbestimmten maximalen Abtragrate eine aktuelle Abtragrate einstellenden Regler (20), wobei die Abtragraten jeweils durch ein aus Sonotroden-Amplitude einerseits und Spülrate oder Unterdruck andererseits bestehendes Wertepaar oder ein aus Sonotroden-Amplitude, Spülrate und Unterdruck bestehendes Wertetripel bestimmt sind.

2. Ultraschallsystem nach Anspruch 1, **gekennzeichnet durch** eine Eingabeeinrichtung (10) zur Auswahl eines vorbestimmten Gewebetyps aus einer Gruppe vorbestimmter Gewebetypen.

3. Ultraschallsystem nach einem der vorhergehenden Ansprüche, **dadurch gekennzeichnet, dass** der vorbestimmte Gewebetyp durch dessen Elastizitätsmodul bestimmt ist.

4. Ultraschallsystem nach einem der vorhergehenden Ansprüche, **dadurch gekennzeichnet, dass** die Stellgröße des Reglers (20) jeweils mit den Regelgrößen Sonotroden-Amplitude, Spülrate und/oder Unterdruck monoton verknüpft ist.

5. Ultraschallsystem nach einem der vorhergehenden Ansprüche, **dadurch gekennzeichnet, dass** die Regelgrößen Sonotroden-Amplitude, Spülrate und Unterdruck jeweils einer eigenen monotonen Funktion folgen.

6. Ultraschallsystem nach einem der vorhergehenden Ansprüche, **gekennzeichnet durch** einen die Stellgröße des Reglers (20) auf eine vorbestimmte Abtragrate begrenzenden Schalter (30), wobei die vorbestimmte Abtragrate niedriger als die vorbestimmte maximale Abtragrate ist.

7. Verfahren zum Betreiben eines ein Ultraschall-Instrument mit einer Sonotrode und einem piezoelektrischen Wandler, einen den Wandler antreibenden Ultraschall-Generator und eine mit dem Ultraschall-Instrument kommunizierende Einrichtung, die zum Zuführen einer Spülflüssigkeit zum Ultraschallinstrument mit einer vorbestimmten Spülrate und/oder zum Bewirken eines vorbestimmten für die Absaugung von mittels dem Ultraschall-Instrument fragmentiertem Gewebe eingerichteten am Ultraschall-Instrument anliegenden Unterdrucks eingerichtet ist, aufweisenden Ultraschallsystems für die Ultraschallchirurgie mittels
- Erzeugen einer vorbestimmten Sonotroden-Amplitude,
- Zuführen einer Spülflüssigkeit zum Ultraschallinstrument mit einer vorbestimmten Spülrate und/oder
- Bewirken eines vorbestimmten für das Absaugen von mittels dem Ultraschall-Instrument fragmentiertem Gewebe eingerichteten am Ultraschall-Instrument anliegenden Unterdrucks,
zum Bewirken einer vorbestimmten Abtragrate,
**dadurch gekennzeichnet, dass**
die Sonotroden-Amplitude und die Spülrate und/oder der Unterdruck mittels eines einzigen Stellglieds in Abhängigkeit eines vorbestimmten Gewebetyps miteinander verknüpft sind.

8. Verfahren nach Anspruch 7, **dadurch gekennzeichnet, dass** der vorbestimmte Gewebetyp durch dessen Elastizitätsmodul bestimmt ist.

9. Verfahren nach einem der Ansprüche 7 und 8, **dadurch gekennzeichnet, dass** der vorbestimmte Gewebetyp vom Anwender ausgewählt ist.

10. Verfahren nach einem der Ansprüche 7 bis 9 **gekennzeichnet durch** Einstellen des Stellglieds auf ein aus Sonotroden-Amplitude und Spülrate und/oder Unterdruck bestehendes Wertepaar oder Wertetripel, das eine vorbestimmte Abtragrate bewirkt, die zwischen einer vorbestimmten minimalen Abtragrate und einer vorbestimmten maximalen Abtragrate liegt.

11. Verfahren nach einem der Ansprüche 7 bis 10, **dadurch gekennzeichnet, dass** die Sonotroden-Amplitude und die Spülrate und/oder der Unterdruck mit dem Stellglied jeweils monoton verknüpft sind.

12. Verfahren nach einem der Ansprüche 7 bis 11, **dadurch gekennzeichnet, dass** die Sonotroden-Amplitude, die Spülrate und/oder der Unterdruck über den Regelbereich des Stellglieds jeweils einer eigenen monotonen Funktion folgen.
